(19) Europäisches Patentamt
European Patent Office
Office européen des brevets

(11) **EP 1 284 639 B1**

(12) **EUROPEAN PATENT SPECIFICATION**

(45) Date of publication and mention of the grant of the patent:
**30.07.2008 Bulletin 2008/31**

(51) Int Cl.:
***A61B 5/00*** (2006.01)

(21) Application number: **00972206.7**

(22) Date of filing: **16.10.2000**

(86) International application number:
**PCT/US2000/028610**

(87) International publication number:
**WO 2001/085021 (15.11.2001 Gazette 2001/46)**

(54) **SYSTEM AND METHOD FOR DETERMINING THE PROBABLE EXISTENCE OF DISEASE**

SYSTEM UND VERFAHREN ZUR FESTSTELLUNG DER WAHRSCHEINLICHEN EXISTENZ EINER KRANKHEIT

SYSTEME ET PROCEDE PERMETTANT DE DETERMINER L'EXISTENCE PROBABLE D'UNE MALADIE

(84) Designated Contracting States:
**AT BE CH CY DE DK ES FI FR GB GR IE IT LI LU MC NL PT SE**

(30) Priority: **08.05.2000 US 202732 P**

(43) Date of publication of application:
**26.02.2003 Bulletin 2003/09**

(73) Proprietor: **Medoctor, Inc.**
**Napa, CA 94558 (US)**

(72) Inventors:
• **Schmitt, Armand J. c/o Medoctor, Inc.**
**Suite 212, Sonoma, CA 95476 (US)**

• **Aguilera, Jeffrey c/o Medoctor, Inc.**
**Suite 212, Sonoma, CA 95476 (US)**

(74) Representative: **McLeish, Nicholas Alistair**
**Maxwell et al**
**Boult Wade Tennant**
**Verulam Gardens**
**70 Gray's Inn Road**
**London WC1X 8BT (GB)**

(56) References cited:
**US-A- 5 660 183      US-A- 5 778 882**
**US-A- 5 935 060      US-A- 6 022 315**

EP 1 284 639 B1

## Description

The description which follows is for a system and method for determining the existence probability of disease.

## Technical Field

The present invention generally relates to the use of database systems for the purpose of determining the existence probability of disease, and more specifically, to the use of such systems by health care providers to more efficiently and accurately determine the existence probability of disease.

## Background Art

The present health care system for processing patients from patient intake through physician diagnosis and treatment is well-known. Health care providers, such as hospitals, medical clinics or health maintenance organizations, provide a multi-step process involving the patient and several different health care professionals. Typically, the patient is initially directed by a receptionist to sign in, and if it is the patient's first visit to the health care provider, to complete a medical history questionnaire, including a description of the patient's symptoms, known drug interactions, and allergies. After waiting in a lobby or reception area for an indeterminate amount of time, the patient is usually next seen by a nurse or nurse practitioner who escorts the patient to a consultation or treatment room where the nurse takes and records the patient's vitals, usually his or her weight, temperature and blood pressure. The nurse will normally ask the patient about the patient's symptoms and records the patient's answers on a medical chart. At this point, the patient will normally be asked to wait in the consultation room until the patient's treating physician is able to see the patient.

Before seeing the patient, the physician will usually look at the nurse's notes on the patient's chart. During the physician's consultation with the patient, the doctor will again ask the patient to describe his or her symptoms, again writing the patient's answers on the chart. The physician will then normally conduct a physical examination of the patient and, ultimately, diagnose the patient's disease or condition. If the physician is unsure of the diagnosis, he or she may recommend that the patient see a specialist to make a diagnosis, and/or may order that certain laboratory tests be performed. Once the diagnosis is made, the physician prescribes a course of treatment or therapy.

There are several aspects of the above-described health care system which contribute to its deficiencies and limitations. For example, after the patient has checked in with the receptionist and possibly completed a medical questionnaire, the patient typically waits for several minutes before actually seeing a nurse. Naturally, during this waiting period, no information about the patient's current medical condition is being obtained. Further, the patient is repeatedly asked, by one or more nurses and ultimately by the treating physician, to describe his or her symptoms. Each time the health care practitioner records the patient's responses on the patient's chart. More thorough practitioners may also ask the patient to describe his or her personal and family medical histories, again maybe more than once. Obtaining information about the patient's personal and family medical histories in this fashion is rife with potential inaccuracies due to its reliance on the patient's memory.

Inaccuracies in a patient's personal and family medical histories may, in particular, lead to an incorrect diagnosis by the treating physician. An incorrect diagnosis may also result from the physician's inadvertent failure to consider the significance of the patient's symptoms to all possible diseases, especially those symptoms that indicate disease or condition about which the physician may not have much experience or familiarity. In this regard, a helpful diagnostic tool would be something that would prompt the physician to recommend that the patient see a specialist who had the requisite experience or to suggest to the treating physician that certain, possibly new, laboratory tests be carried out before a final diagnosis is made.

The prior art comprises document US-A-5 778 882 describing a health monitoring system tracking a patient's state of health by periodically measuring and recording physiological data from sensors in contact with the patient's body. The data collected is not specifically related to a particular medical condition.

The prior art further comprises document US-A-6 022 315 describing a system and method for providing computerized knowledge-based medical diagnostic and treatment advice (MDATA) to a patient. The method comprises selectively executing a plurality of medical complaint algorithms based on the information received by the patient. An embodiment thereof utilizes medical diagnostic scripts following an evaluation process wherein the patient is prompted to describe a complaint by anatomic system. At a later stage the system proceeds to a complaint menu and recites a list of algorithms from which the patient chooses one. The computer then presents a series of initial problem screening questions. Based on the answers, the computer reorders a list of complaint causes, initially ranked in decreasing frequency of occurrence in the general population, to a new order of decreasing probability in the patient seeking consultation. In that order, the system asks confirmation questions related to the complaint cause, leading to the evaluation of a probability of confirmation of said complaint cause. A complaint cause is confirmed by having a probability of confirmation above a threshold. Elicited complaint causes receive a score pertaining to the answers given by the patient, and a diagnosis of the causes related to the chosen complaint is provided in a list of decreasing score, i.e. probability, for the patient.

Accordingly, it is the purpose and goal of the present invention to overcome the inadequacies and limitations of the current health care system for processing patients from patient intake through physician diagnosis and treatment.

**Disclosure of Invention**

According to the present invention there is provided an interactive computer system and computer program product and method of operating the interactive computer system as claimed in the appended claims.

The present invention is a new and unique computer implemented and interactive system, the use of, a computer program product and the use thereof for obtaining a patient's symptoms, and other indications and predispositions for disease, which are then utilized to determine the existence probability of disease. The patient's symptoms and other information are inputted by a patient into the programmed computer system in response to computer inquiries that are generated by a program stored within the memory of the computer system. The patient accesses the computer system by using a computer input and display device, located at the facility of a health care provider, such as a hospital, medical clinic or Health Maintenance Organization.

Once the patient has gained access to the computer system, the system's program generates a series of inquiries which are displayed on the patient's display device. Initially the patient is asked to identify the Body Part which the patient believes is causing or is related to the patient's physical or mental problem. Next, the patient is prompted to input the patient's vitals: weight, temperature, and blood pressure. The patient is also asked to indicate the degree of pain, if any, being experienced. Once the patient has identified a Body Part, the system's program retrieves from a Disease/Symptom database all of the diseases and their related symptoms, indications, and predispositions which are related to the Body Part.

The Disease/Symptom database is created, in part, from a publicly available list of diseases which contains virtually all of the presently known human diseases, with each disease being identified by a unique alphanumeric code pursuant to the International Classification of Diseases ("ICD" code). The total number of persons who have been diagnosed with each disease is also tracked and continually updated as new diagnoses are confirmed. Creation of the Disease/Symptom database is generally completed by associating a numerical value for each disease equal to the number of times each disease has been found to exist in a patient (referred to herein as the "Disease Person Count"), and associating a list consisting of each physical symptom (*e.g.*, pain), predisposition (*e.g.*, heart disease) and other indications of disease (*e.g.*, toxic exposure, low white cell count) (hereinafter individually referred to for convenience as a "Symptom," or collectively as "Symptoms") to each disease. Each Symptom associated to a specific disease is also assigned a numerical value (referred to herein as the "Symptom Person Count") equal to the Disease Person Count for that disease. Lastly, each Symptom and its associated Person Symptom Count is associated to a predetermined Body Part.

After all Symptoms are identified as pertaining to a Body Part, the system's program selects the Symptom, out of the list of Symptoms associated to the Body Part, which has the highest probability of indicating a disease related to the Body Part selected by the patient. This probability is determined by the program by comparing the Symptom with the highest Symptom Person Count to the sum of all Symptom Person Counts for the Body Part. After the most probable Symptom is identified, the program generates a question which asks the patient, depending upon which Symptom has been identified, whether he/she has a certain physical symptom, whether he/she or anyone in his/her family has, or has ever had, a specific disease, and whether he/she has recently been exposed to a certain environmental condition. After the patient answers the question, the program updates the probability that the patient has one of the diseases contained in the entire Disease/Symptom database. The existence probability of each disease in the database is updated due to the fact that a condition, namely the existence or nonexistence of a Symptom, has been indicated by the patient.

If the patient responds to an inquiry by affirming that he/she has the Symptom, the system's program then uses Bayesian statistics to update or incrementally increase the existence probability of each diseases in the Disease/Symptom database which is indicated by the patient's symptom. On the other hand, if the patient does not have the Symptom, then the program updates the Disease/Symptom database by either incrementally increasing or decreasing the existence probability of each diseases which does not exhibit the Symptom. The program then compares the disease probability for each disease in the Disease/Symptom database to a predetermined threshold value, and for each disease having a probability equal to or greater than the threshold, its Symptoms are added to the list of Symptoms initially associated to the Body Part previously identified by the patient, assuming the symptoms are not already listed. After the probability of each disease is updated, the Symptom is removed from the Disease/Symptom database. The system's program then selects the Symptom from the list of Symptoms associated to the Body Part which has the highest patient Symptom count of all remaining Symptoms. Then, the program repeats the process of generating a patient question based upon the Symptom, updating the existence probability of each disease in the Disease/Symptom database, comparing the disease probabilities to the predetermined threshold value, and removing the Symptom from the database. This repetitive process continues until all of the Symptoms from the Disease/Symptom database, which were originally identified to the patient's Body Part, are removed from the database. The system's program next displays and ultimately generates a disease probability report or "pre-diagnosis" report in which the patient's most probable disease is identified, along with a next most probable disease and other less probable diseases. The pre-diagnosis report is then made available to the health care practitioners and to the patient's treating or primary care physician.

It is anticipated that the patient's nurse or other health care practitioner will review the pre-diagnosis report to determine whether the information inputted by the patient into the system is accurate and whether the patient has adequately responded to the computer generated interview questions which identify the patient's Symptoms. It is also anticipated that the pre-diagnosis report will then be given to the treating physician before he/she sees the patient. In this manner, the physician will be able to obtain a comprehensive medical history and present condition of the patient, and most importantly a pre-diagnosis report of the patient's most probable disease and other less probable diseases. After reviewing the report, the physician will have obtained comprehensive medical information about the patient and the patient's likely disease, and as a result, will be thoroughly prepared to personally examine the patient. Once the physician completes the examination, he/she will then be fully informed about the patient and the patient's medical condition, and will be thoroughly prepared to reach a diagnosis of the patient's disease or condition. If the physician's own diagnosis is the same as the diagnosis predicted in the pre-diagnosis report, the doctor will be extra confident that he or she has correctly diagnosed the patient's actual disease. If the physician's diagnosis is different from the pre-diagnosis, the physician may be prompted to advise the patient to obtain a second opinion. The pre-diagnosis report may also prompt the treating physician to consider other, less probable, diseases about which he/she may not be completely familiar. In such cases, the treating physician may want to recommend that the patient see a specialist to evaluate the patient before a final diagnosis is made.

Another advantage of an embodiment of the present invention is its ability to continually improve the accuracy of the computer system's determination of the existence probability of disease. Each time the system diagnoses a disease, which is confirmed by the treating physician and/or a specialist to be the patient's actual disease, the disease and its associated Symptoms are updated in the Disease/Symptom database. As a result, the more times the system is used to generate a pre-diagnosis, the more statistical information is obtained about the number of patients who have been diagnosed with a disease. As the quantity of statistical information increases, the reliability and accuracy of the determination of the existence probability of disease similarly increases.

**Brief Description of Drawings**

FIG. **1** is a block diagram and schematic which illustrates the overall architecture of the present invention

FIG. **2** is a schematic which illustrates the organizational structure of the Disease/Symptom database.

FIG. **3** is a block diagram and schematic which illustrates a patient's interaction with the application processor.

FIG. **4** is a diagram of a computer screen which the patient uses to input his or her vitals.

FIG. **5** is a diagram of a computer screen which the patient uses to identify a Body Part.

FIG. **6** is a diagram of a computer screen which the patient uses to indicate the existence or nonexistence of a Symptom.

FIG. **7** is a table which identifies "Question Phrases" and "Template Codes" and illustrates their relationship.

FIG. **8** is a block diagram and schematic which illustrates the manner in which the processor generates a question to be presented to the patient.

FIG. **9** is a flow chart which illustrates the manner in which the processor determines the existence probability of disease.

FIG. **10** is a diagram of a pre-diagnosis report.

**BEST MODE FOR CARRYING OUT THE INVENTION**

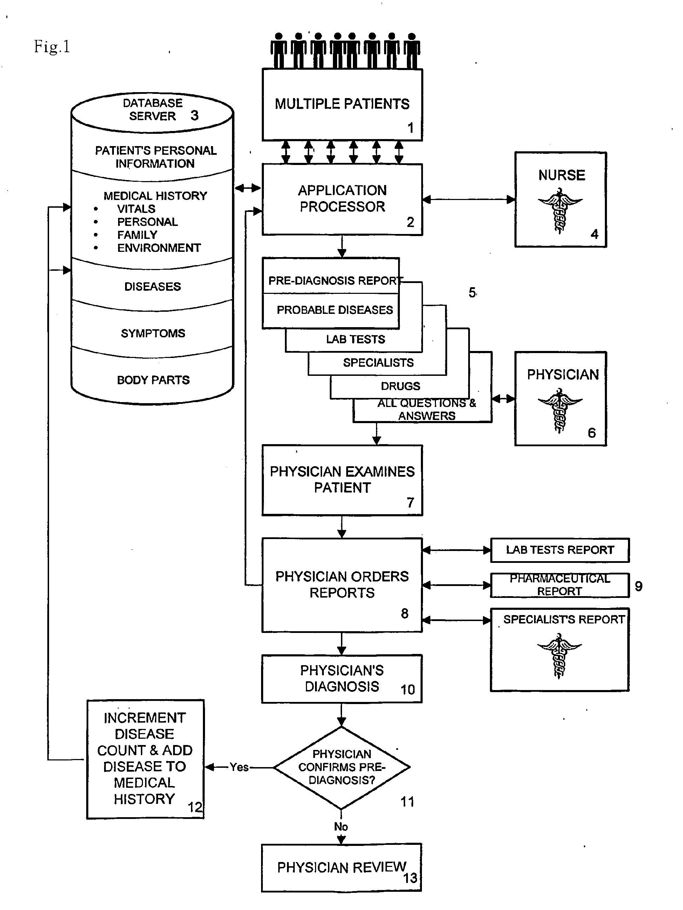

In a preferred embodiment of the present invention, the computer implemented and interactive system and method for determining the existence probability of disease is most readily utilized by a health care provider such as a hospital, medical clinic, or Health Maintenance Organization. In this regard, FIG. **1** illustrates the general architecture of the present invention which enables health care providers to process patients more efficiently and effectively during the patient intake phase, to more accurately and comprehensively obtain and record the patient's symptoms, and to assist the treating physician in reaching a correct diagnosis. Step 1 illustrates that the present invention allows the health care provider to simultaneously process a plurality patients, who are each connected, at step 2, to an application processor, comprising one or more programmed computer servers. The server may be programmed by using a program storage device, such as a CD-ROM, containing computer readable program code. An example of an acceptable CD-ROM is a Hewlett Packard CD-ROM, bearing model number hp 48x max IDE CD-ROM and product number D944A, having a storage capacity of 650 MB.

The patients are prompted by the application processor to input information relating to their medical condition and history and to answer processor generated questions indicating whether they have a specific Symptom which information is, in turn, stored at step 3 in the memory of a database server. The database server also stores the "Disease/Symptom" database containing lists of all diseases, all Symptoms, and all Body Parts. At step **4,** a nurse or nurse practitioner reviews the medical information inputted by the patient and determines whether the patient has adequately responded to the questions about their Symptoms, and consults with the patient, if necessary, to correct any errors or inconsistent data. Once the patient's medical information has been verified, the programmed application processor uses the data stored in the database server to calculate, the possible existence of all diseases. At step **5,** a pre-diagnosis report is generated which identifies the patient's most probable disease and other less probable diseases. The report may also recommend that certain laboratory tests be performed, that a specialist be consulted, and that certain drugs be considered by the physician. And, at step **6,** the pre-diagnosis report is presented to the physician, providing the doctor with an opportunity, before seeing the patient. to become thoroughly informed about the patient's medical condition and history and most importantly, informed about the patient's most probable disease and other less likely diseases which should be considered. At step **7,** the patient's physician examines the patient and may make a preliminary diagnosis. At steps **8** and **9,** the physician may order and receive a laboratory test report, a specialist's report and/or a pharmaceutical report. If a laboratory test is obtained which further describes the patient's physical condition (*e.g.*, patient has a low white cell count), the processor also at step **8** prompts the physician to affirm that the condition exists and the processor recalculates the existence probability of all diseases and a revised pre-diagnosis report is generated. Finally, at step **10,** the physician makes a final diagnosis based upon the information contained in the pre-diagnosis report, the doctor's examination, and upon any laboratory test results and/or the result of any specialist's report.

After the treating physician makes a final diagnosis, the physician is asked at step **11** to respond to an inquiry at the end of the pre-diagnosis report to either confirm that the pre-diagnosis was correct or to indicate that the pre-diagnosis was not correct. If the physician confirms that the pre-diagnosis correctly identified the patient's actual disease, the application processor updates the Disease/Symptom database at step **12** by incrementing the Patient Disease Count by one (1) and adding the disease to the patient's medical history stored in the database server. If the accuracy of the pre-diagnosis report is not confirmed, the physician at step **13** may further review the pre-diagnosis report, re-examine the patient, obtain additional laboratory tests, seek further analysis by a specialist and/or report to the health care provider of the inability to confirm the pre-diagnosis.

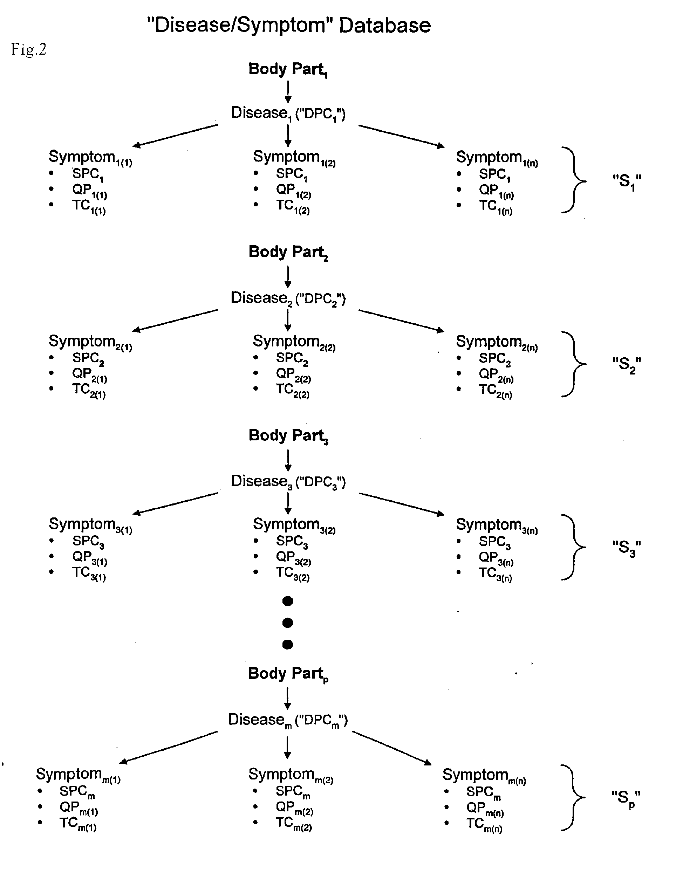

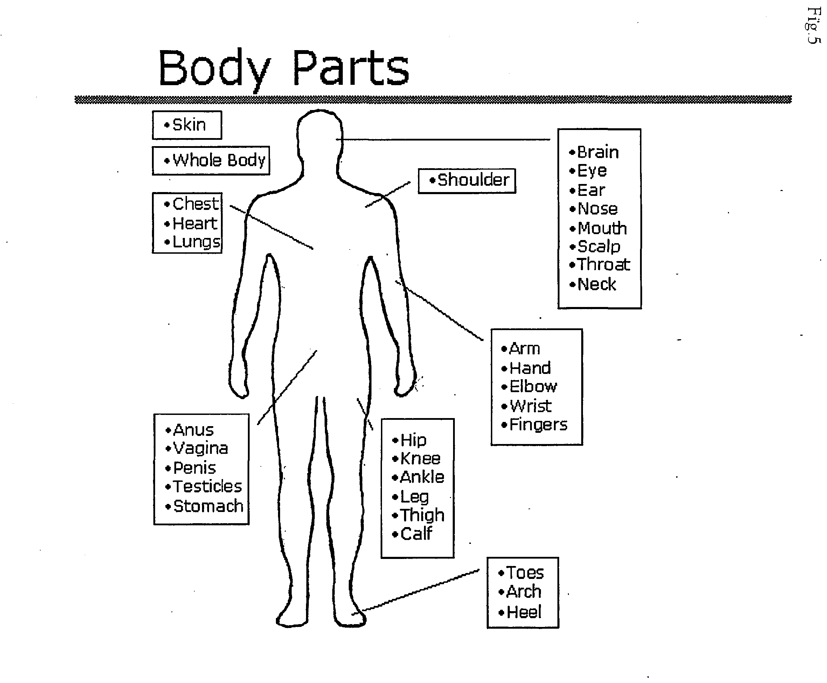

FIG. **2** generally illustrates the organizational structure and content of the Disease/Symptom database. Initially, the database identifies a predetermined number of human Body Parts, which are depicted in FIG. **2** as Body Part$_1$, Body Part$_2$, Body Part$_3$ ... Body Part$_p$. The identity of each Body Part is as depicted in FIG. **5** and will be described further in connection with the subsequent description of that figure.

Next, the Disease/Symptom database associates with each Body Part virtually all the known diseases which are generally associated to that Body Part. The organization of each disease within the Disease/Symptoms database is shown in FIG. **2** as Disease$_1$, Disease$_2$, Disease$_3$ ... Disease$_m$. Although the figure only illustrates a single disease in relation to a Body Part, it is likely that several diseases may be associated in the database to a given Body Part. In addition, each disease is associated with a "Disease Person Count" ("DPC"), illustrated in FIG. 2 as DPC$_1$, DPC$_2$, DPC$_3$ ... DPC$_m$, which is a numerical value equal to the number of persons who have been confirmed to exhibit a specific disease. The identity of each disease is initially obtained from a publicly available list of diseases ("public database"), maintained and reported by the *World Health Organization* which contains virtually all of the presently-known human diseases. This public database assigns a unique, alphanumeric code to each disease pursuant to a scheme called the "International Classification of Diseases" ("ICD" code). Similarly, the present invention uses the ICD code to identify each disease in the Disease/Symptom database. The initial Disease Person Count of each disease has been obtained from other resources and publicly reported information which have confirmed that a certain number of persons exhibit each disease. This information has been compiled to establish a Disease Person Count for each disease in the Disease/Symptom database. This Disease Person Count information is also used to establish the initial existence probability of each disease by determining the quotient of the Disease Person Count for each disease divided by the total Disease Person Count for all diseases. In this manner, the present invention uses the *World Health Organization* public database and other publicly available information about the frequency of diseases to partially initialize the Disease/Symptom by providing a list of known diseases and a count of the number of persons who have exhibited each disease and the initial existence probability of each disease.

The Disease/Symptom database is further organized by associating with each disease a list of Symptoms which are known to indicate the disease or to predispose the patient to having the disease. In this regard, as discussed above in the Disclosure of Invention, "Symptoms" are defined herein to include the patient's physical symptoms (*e.g.*, pain and stiffness), the patient's physical condition which is revealed by the physician's examination and/or laboratory tests (*e.g.*, a heart murmur and a low white blood cell count), the patient's predisposition to disease (*e.g.*, prior diseases of the patient and the patient's family), and environmental conditions which are known to be a factor in causing certain diseases. As further illustrated in FIG. 2, $Disease_1$ is associated with $Symptom_{1(1)}$, $Symptom_{1(2)}$ ... $Symptomm_{1(n)}$. In addition, the set of all Symptoms associated with each specific Body Part, is identified as set "S". More specifically, set $S_1$, $S_2$, $S_3$ ... $S_P$ is each associated with $Body Part_1$, $Body Part_2$, $Body Part_3$ ... $Body Part_p$, respectively. The Disease/Symptom database also associates with each Symptom a "Symptom Person Count" ("SPC") which is a numerical value equal to the Disease Person Count for the disease which is related to that Symptom and Body Part. For example, in FIG. **2**, $SPC_1$ is the Symptom Person Count for $Symptom_{1(1)}$, $Symptom_{1(2)}$... $Symptom_{1(n)}$, and $SPC_1$ equals $DPC_1$. As a result, the database is organized such that each Symptom Person Count is equal to the Disease Person Count for a given Body Part.

Lastly, the Disease/Symptom database associates with each Symptom a "Question Phase" ("QP") and a "Template Code" ("TC") which are,used by the application processor to generate a question to be presented to the patient requesting that the patient indicate whether: 1) the patient has a physical symptom; 2) the patient has ever had a specific disease; 3) any member of the patient's family has had a specific disease; and 4) the patient has been exposed to an environmental condition.

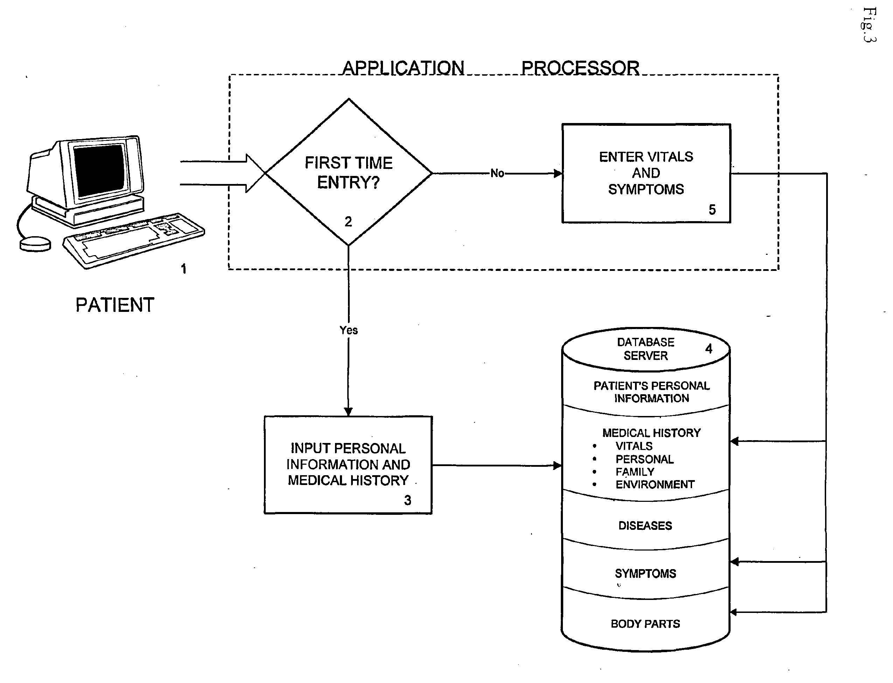

FIG. **3** illustrates in more detail the manner in which each patient enters his or her medical information using a computer monitor input and display device. Immediately, upon the patient's arrival at the health care provider's facility, he or she is directed to a private computer monitor area where the patient will be prompted by the system's application server to input his or her medical information. In a preferred embodiment, the patient's health care provider furnishes the patient with an identification card which the patient, as shown at step **1,** swipes through a card reader connected to the application processor. If the processor determines that the patient is authorized, the patient's computer monitor is activated by the processor. Initially, at step **2,** the processor determines whether the patient is entering medical data into the system for the first time. If the patient is a first time user, the processor prompts the patient at step **3** to enter their personal and family medical history by responding to questions presented to the patient on the monitor screen. Preferably, the monitor screen provides "touch-sensitive" radio button technology which allows the patient to respond to an inquiry by merely touching a radio button to indicate in the affirmative. Each time the patient indicates that he or she or a family member has had a certain disease, the processor at step **4** stores the disease in the database server as part of the patient's personal or family medical history. Similarly, if the patient identifies any recent environmental exposures, the information is stored in the database server.

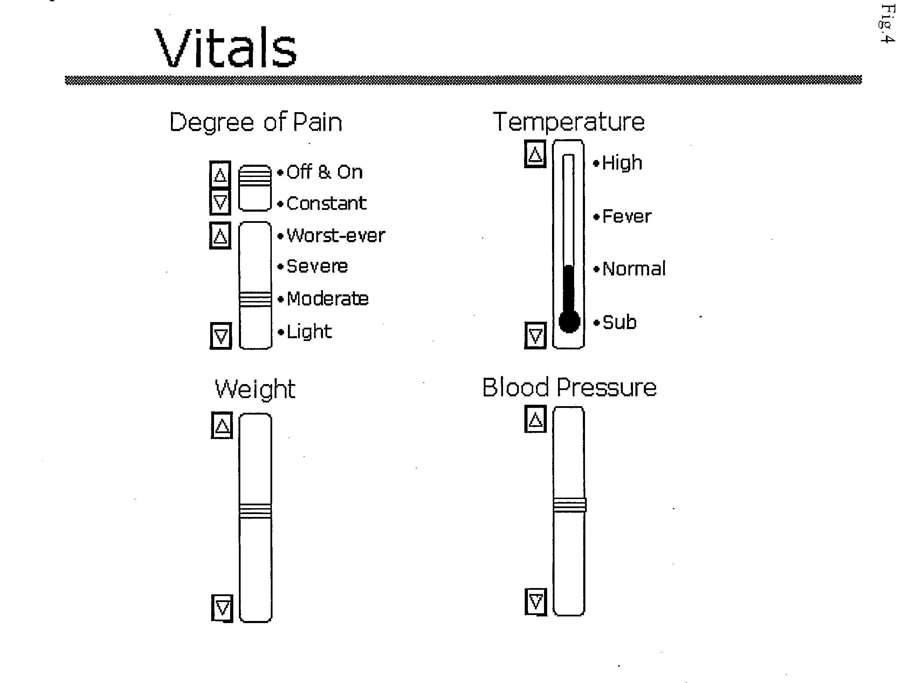

After the patient has completed entering his or her medical history or if the patient is not a first time user, the application processor at step 5 prompts the patient to enter his or her vital information and personal symptoms. The processor displays a "Vitals" screen as illustrated in FIG. **4.** One of the vitals displayed to the patient is a degree of pain indicator **1** and the patient is prompted to touch that portion of the display which corresponds to his or her level of pain. In the patient's private computer monitor area, each patient is also provided with sensory devices which the patient uses to obtain his or her temperature, weight and blood pressure. Once these values are obtained, the patient is again prompted to input each value into the system by touching the temperature display **2,** weight display 3 and blood pressure display **4,** and the Vitals are stored by the processor in the Disease/Symptom database. In another embodiment, each sensory device is connected to the processor which automatically stores the information in the Disease/Symptom database.

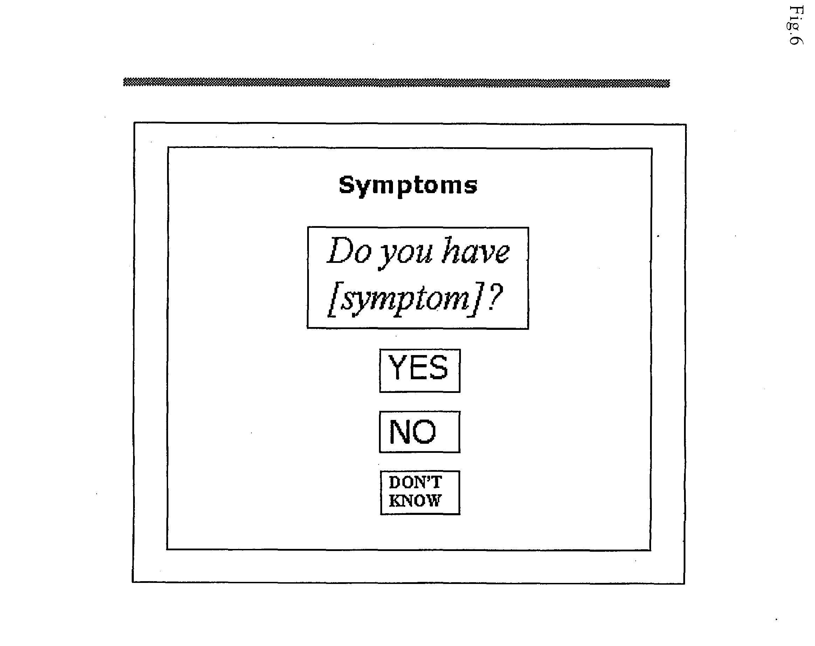

Once the patient has completed entering his or her Vitals, the processor displays on the patient's screen, as illustrated in FIG. **5,** a human figure depicting a plurality of Body Parts, with each Body Part being further identified by a textual description and a radio button. This important aspect of the invention prompts the patient to identify that part (or parts) of his or her body which the patient believes is associated with the personal physical symptom or symptoms being experienced by the patient. After the patient identifies a Body Part by touching the radio button adjacent to said Body Part, the processor initiates another interactive process, as depicted in FIG. 6, in which the patient is asked a series of questions concerning the Symptoms related to the identified Body Part.

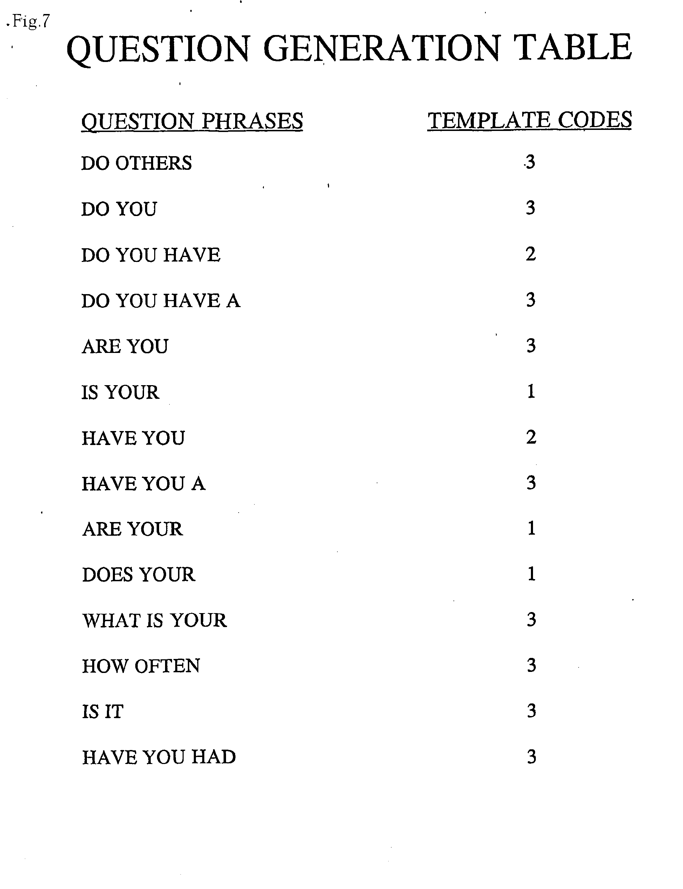

Before describing the patient's interaction with the system as illustrated in FIG. 6, FIG.'s 7 and 8 are described in order to explain the manner in which the application processor generates each question which is presented to the patient. As shown in FIG. 7, the present invention also stores in the database server a table of "Question Phrases" and "Template Codes," either 1, 2, or 3, associated with each phrase. Further, each Questions Phrase ("QP") and its associated Template Code ("TC") is also associated to a specific Symptom stored in the "Disease/Symptom" database as described above and shown in FIG. 2.

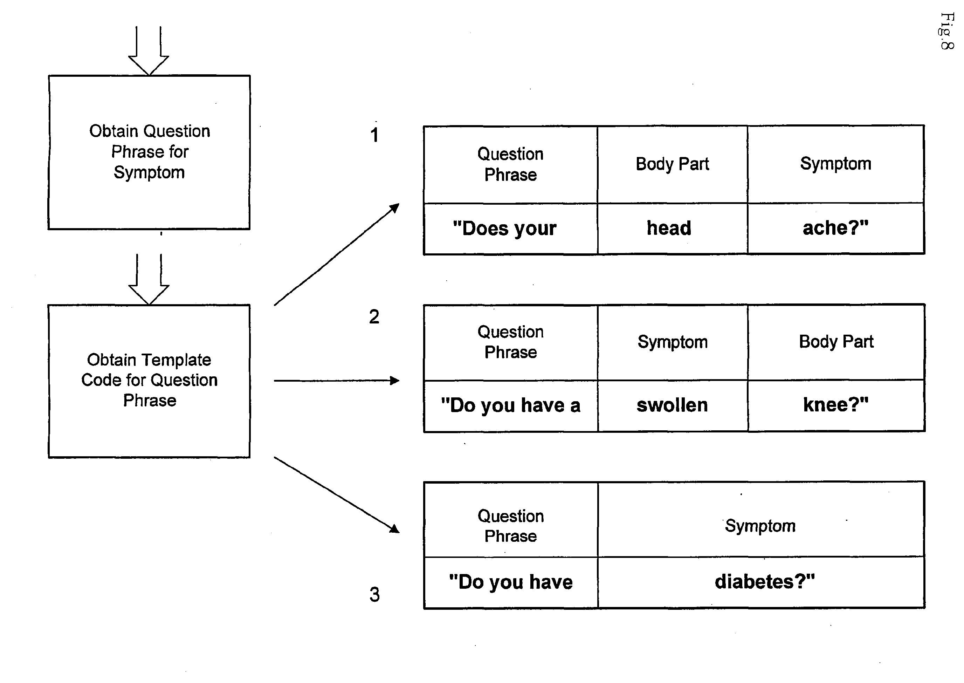

The first step performed by the application processor in creating a question is to identify the Symptom out of the set of "S" Symptoms (*i.e.*, all Symptoms related to the Body Part identified by the patient) which has the largest Symptom Person Count, which means that the Symptom has the highest frequency out of all Symptoms in the set "S" related to a Body part. Next, as shown in FIG. **8,** the processor identifies the Question Phrase and Template Code associated with the identified Symptom. Based upon the Template Code identified, the processor selects either Template 1, 2, or 3. If Template 1 is selected, the sentence is constructed by beginning the sentence with the Question Phrase, placing the Body Part, previously identified by the patient, next, and the Symptom last (*e.g.*, "Does your head ache"). If Template 2 is selected, the order of arrangement is Question Phrase, Symptom and Body Part (e.g., "Do you have a swollen knee"). And, if Template 3 is identified, the order is Question Phrase and Symptom (e.g., "Do you have diabetes"). Finally, the completed question is presented to the patient by the processor as shown in FIG. 6, and in a preferred embodiment the patient is prompted to respond by touching the screen to respond to the question by answering "YES," "NO" or "DON'T KNOW," indicating that: 1) he or she has or does not have the symptom or the disease; 2) that a family member has or has had the disease; or 3) he or she has or has not been exposed to an environmental condition.

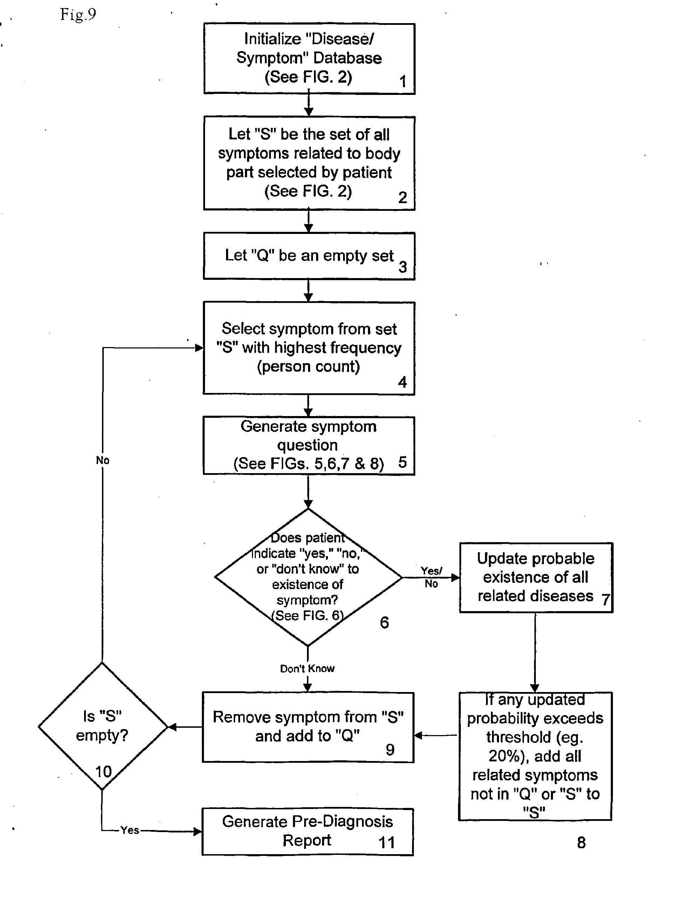

At this point the application processor utilizes the patient's answer, as illustrated in FIG. 9, to determine the existence probability of all diseases stored in the database server. In step 1 of FIG. 9, the processor initializes the Disease/Symptom database. This initialization process is the same as the process described above in connection with FIG. 2, in which the Disease/Symptom database is created by establishing a predetermined number of Body Parts, associating a list of diseases to each Body Part, associating a list of Symptoms to each disease, and establishing the initial probability of each disease in the database.

At step **2,** the processor defines a set "S" to be all Symptoms related to a Body Part selected by the patient (see also FIG. 2), and at step **3,** a empty set "Q" is defined. At step **4,** the processor selects the Symptom out of all possible Symptoms ("S") identified in step 2 which has the highest frequency or - Symptom Person Count for the Body Part.

Once the Symptom with the highest Symptom Person Count has been identified, the processor at step 5 generates and displays a questions to the patient as described above in connection with FIG.'s 5, 6, 7, and 8. If at step 6, the patient indicates by answering "YES" that he or she, or a family member, has or has had the Symptom (*i.e.*, personal physical symptom or disease) or has been exposed to the Symptom (*i.e.*, environmental condition), the processor proceeds at step 7 to update or incrementally increase the existence probability of all diseases in the Disease/Symptom database which are indicated by the patient's Symptom. On the other hand, if the patient indicates that he or she does not have the Symptom by answering the question "NO", the processor updates at step 7 the Disease/Symptom database by either incrementally increasing or decreasing the existence probability of each diseases which is not indicated by the Symptom.

In either event, the probabilities are updated in accordance with Bayesian statistics:

$$\Pr\{D|S\} = \Pr\{D\}\,\frac{\Pr\{S|D\}}{\Pr\{S\}}$$

Where Pr {D|S} is the probability that disease D exists given the fact the patient has or does not have Symptom S; and Pr {D} is the probability that disease D exists without any condition.

When the patient indicates that he/she has a Symptom, Pr {S|D}/Pr {S} is equal to:

$$\frac{P_{ij}}{S_j}$$

Where $P_{ij}$ is the conditional probability that Symptom **j** indicates disease **i**, and $S_j$ is the unconditional probability of Symptom **j**.

Thus, for ease disease **i** which is indicated by Symptom **j**, the existence probability of the disease is calculated as follows:

$$\Pr_{(after)}\{D_i\} = \Pr_{(before)}\{D_i\}\frac{P_{ij}}{S_j}$$

Where

$$P_{ij} = \frac{\text{Symptom Person Count for Symptom j}}{\text{Total Symptom Person Count for Disease i}}$$

$$S_j = \frac{\underline{\text{Symptom Person Count for Symptom } \mathbf{j}}}{\text{Total Symptom Person Count for Symptom } \mathbf{j} \text{ for all diseases } \mathbf{i}}$$

If the patient indicates that he/she does not have the symptom, Pr {S|D}/Pr {S} is determined as follows:

$$\frac{1 - P_{ij}}{1 - S_j}$$

At step **8,** the existence probability of all diseases is compared to a predetermined threshold value (**e.g.,** a 20% probability) and for all qualifying diseases having a probability equal to or greater than the threshold, those of their Symptoms are added to the set of symptoms "S" which are not already listed in set "S" or "Q". Then at step 9 the Symptom previously selected at step 4 is removed from the set "S" since the existence or nonexistence of the Symptom has already been used to update all disease probabilities. At the same time, the Symptom and the fact of its existence or non-existence is added to data set "Q," which will be used later when the pre-diagnosis report is prepared, along with a listing of the patient's reported Symptoms.

Returning to step **6,** if the patient does not respond to a question, the program proceeds directly to step **9** where the Symptom is removed from the set "S" and added to "Q" as a Symptom about which the patient did not respond.

Continuing at step **10,** the processor determines whether the set "S" contains any more Symptoms. If it does, the program returns to step **4** and the process just described is repeated. When all Symptoms have been removed from the set "S", the program is complete, and the pre-diagnosis report is generated at step **11** and the report is displayed on a nurse display device and a physician display device.

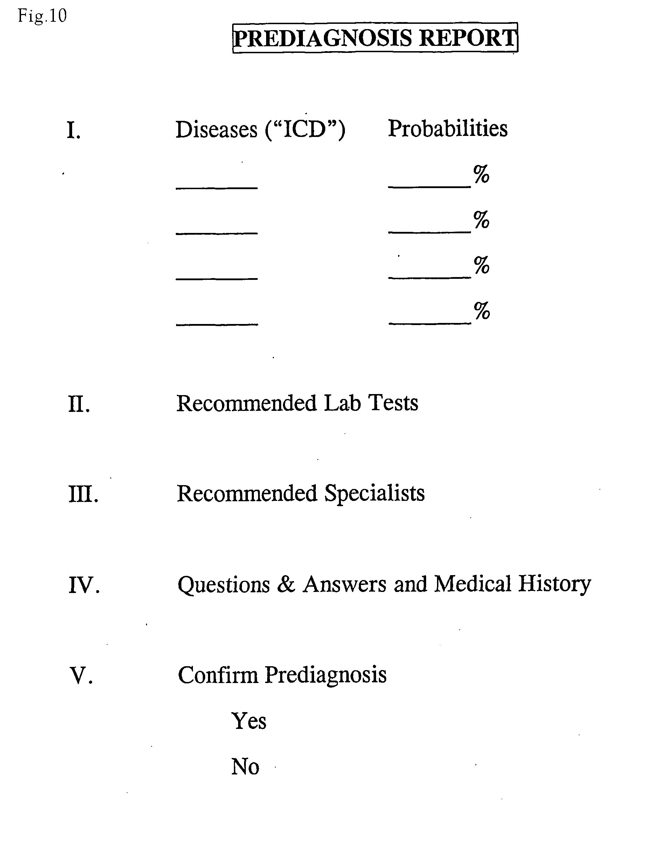

The format of the pre-diagnosis report is illustrated in FIG. 10. In addition to identifying the patient's most probable disease and its "ICD" number, the report delineates the existence probability of all diseases which have met the predetermined threshold value. The report also sets forth any laboratory tests that are recommended based upon the identity of the most probable diseases. The pre-diagnosis report may also recommend that a specialist be consulted if the probability of the most probable disease and the next most probable disease or diseases is within a certain predetermined percentage (**e.g**., 5%). The report further contains a description of the patient's medical history and the patient's family history, and a listing of all questions presented to the patient with each response. If the physician orders laboratory tests and the test results further describe the patient's physical condition, which the present invention treats as another Symptom, the processor prompts the physician to indicate that the condition exists. Once the physician does so by using a physician data entry device such as a touch sensitive screen in connection with the processor, the processor recalculates the existence probability of all diseases based upon the fact that another Symptom was found to exist, and generates and displays to the physician a revised pre-diagnosis report.

Finally, the pre-diagnosis report asks the physician to confirm the pre-diagnosis. In a preferred embodiment, confirmation of a pre-diagnosis shall also include all diseases which are determined to have a 95 % or greater probability. If the physician confirms, based upon his or her own physical examinations and study of any laboratory test results, that the pre-diagnosis report correctly identified the most probable disease as the actual disease, or that one of the diseases identified by the report as part of a sublist of diseases having at least a 95 % probability of existence, was the actual disease, the processor prompts the doctor to so indicate by using the physician's touch screen to answer "YES" that the pre-diagnosis is confirmed and, as illustrated in FIG. **1,** the processor increases the Disease Person Count by one (1), and adds the disease to the patient's medical history. If the doctor is unable to confirm the pre-diagnosis, the physician may want to consider advising the patient to obtain a diagnosis from another physician and/or obtain further laboratory tests and/or additional evaluation from a specialist.

Although the present invention has disclosed as its preferred embodiment the circumstance in which patients input their medical information and Symptoms using a data entry method, such as a touch screen and sensory devices, located at the health care provider's facility, it will be readily understood by those skilled in the art that the present invention includes another embodiment in which patients' may enter their medical information and Symptoms from a remote location, such as their home, which is connected over a network to the application processor located at the health care facility. The data entry method at the remote location may consist of any type of data entry device, such as a monitor and keyboard or a touch screen. And, the network may include a proprietary intranet or the public internet. It must also be understood, however, that in this embodiment, after the patient has inputted his or her medical information, the patient will have to visit the health care provider in order to have a treating physician review the patient's pre-diagnosis report and make a final diagnosis.

Further, it should be apparent that many modifications may be made to the present invention without departing from the essential teachings of the invention. Accordingly, it will be understood by those skilled in the art that, within the scope of the appended claims, the invention may be practiced in embodiments other than those specifically described in this application.

**Industrial Applicability**

The present invention has numerous advantageous industrial applications. Health care costs constitute a significant portion of every country's economy, and those costs are continuing to rise dramatically as the world population continues to grow. In addition, person's who can afford to have health insurance are experiencing significant increases in their insurance premiums.

Health care providers are no longer able to efficiently and effectively process patients from patient intake through physician diagnosis and treatment. Although the Health Maintenance Organization ("HMO") was originally developed to reduce the costs of treatment and insurance, in general, patients have become disenchanted with the HMO's slow and inefficient services. More seriously, patient's are noticing that administrators are increasingly preventing patients from obtaining proper medical care.

The present invention constitutes a significant advantage in the manner in which HMO's and all health care providers process patients. The present invention provides health care providers with the ability to simultaneously process multiple patients and to obtain their vital information without requiring the assistance of a nurse or other health care practitioners. The patient's medical information is stored in a computer database which constitutes a permanent and readily obtained record of each patient. The invention also provides a feature which automatically updates the patient's medical records as further information about the patient's medical condition becomes known. Efficiency is further increased because the patient's current symptoms are obtained electronically and automatically displayed to the patient's nurse and physician. Most significantly, the system of the present invention generates a pre-diagnosis report of the patient's condition or disease before he or she ever sees a doctor. The doctor's efficiency is enhanced because he or she is able to review the pre-diagnosis report to become thoroughly familiar with the patient's condition and the patient's possible diseases that should be considered during the patient's examination.

Another significant aspect of the present invention is that the more the invention is used the more valuable and accurate it becomes. Each time a physician confirms the existence of a disease which was predicted in the pre-diagnosis report, the doctor updates the invention's database of diseases which increase the existence probability of the disease. As the quantity of statistical information increases, the reliability and accuracy of the invention's determination of the existence probability of diseases similarly increases.

**Claims**

1. An interactive computer system comprising:

> a. a programmed computer processor;
> b. a database server having a memory, said database server in electronic communication with the processor;
> c. a plurality of patient data entry and display devices all in electronic communication with the processor; adapted for performing the steps as described in d to s:
> d. prompting a patient, out of a plurality of the patients, to enter his or her vitals into the memory of database server by using one of said patient data entry devices in communication with the processor and to similarly enter the patient's personal medical history, family medical history and exposure to environmental conditions into the database server;
> e. storing a list of Body Parts in the memory of the database server;
> f. storing in said memory, lists of diseases ($Disease_1$,....,$Disease_m$) such that for each stored Body Part a list of diseases comprising at least one disease is stored in association with that stored Body Part, and lists of Symptoms {$Symptom_{1(1)}$,..., $Symptom_{1(n)}$}, ... {$Symptom_{m(1)}$ ,...., $Symptom_{m(n)}$}) such that for each stored disease from any of the stored lists of diseases, a list of Symptoms is stored in association with that disease;
> g. storing in said memory, disease numerical values ($DPC_1$, ..., $DPC_m$) such that for each stored disease from any of the stored lists of diseases, a disease numerical value is stored in association with that disease, each said disease numerical value having a value equal to the number of persons who have been confirmed to exhibit that specific disease;
> h. storing in said memory, symptom numerical values ($SPC_1$,..., $SPC_m$) such that for each stored Symptom from any of the lists of Symptoms, a symptom numerical value is stored in association with that Symptom, each said symptom numerical value that is stored in association with a said Symptom in a said list of Symptoms stored in association with a said disease having a value equal to the disease numerical value ($DPC_1,...,DPC_m$) of that same said disease;

i. identifying with the processor sets of Symptoms ($S_1,...,S_p$), and each Symptom's associated symptom numerical value, such that for each stored Body Part a set of Symptoms ($S_1,...,S_p$) is identified in association with that Body Part, said set of Symptoms comprising all Symptoms associated to that Body Part;

j. calculating the existence probability of each disease in each list of diseases by using the processor to determine the quotient of the disease numerical value of each disease divided by the sum of all disease numerical values for all diseases and storing the results in the memory of the database server;

k. calculating a total symptom numerical value for all identical Symptoms in each list of Symptoms by using the processor to determine the sum of all symptom numerical values for all identical Symptoms in each list, and storing the results in the memory of the database server;

l. displaying to the patient on one of said patient display devices a human figure having a plurality of Body Parts;

m. prompting the patient to identify, using the patient data entry device, a target Body Part, out of all Body Parts displayed, which the patient believes is associated to his or her physical or mental condition, and defining a first iterative dataset (S) initially being the set of all Symptoms associated to the target Body Part, and defining a second iterative dataset (Q) initially being empty;

n. identifying with the processor a target Symptom from the first iterative dataset (S) which has the highest total symptom numerical value and generating a question presented to the patient on the patient display device which asks the patient if the target Symptom is or is not indicated;

o. prompting the patient to respond to the question using the patient data entry device;

p. calculating an updated existence probability $Pr_{(after)}\{D_i\}$ of each disease ($D_i$) in each list of diseases based upon the patient's answer by using the processor to determine said existence probability in accordance with the following formula in accordance with Bayesian statistics:

$$\Pr\{D \mid S\} = \Pr\{D\}\frac{\Pr\{S \mid D\}}{\Pr\{S\}}$$

where D is a disease and S is a Symptom, Pr{...} is an unconditional probability and Pr{...|...} is a conditional probability, by calculating the updated existence probability in case the patient indicates that he/she has the Symptom, using the equation:

$$\Pr_{(after)}\{D_i\} = \Pr_{(before)}\{D_i\}\frac{P_{ij}}{S_j}$$

and in case the patient indicates that he/she does not have the Symptom, using the equation:

$$\Pr_{(after)}\{D_i\} = \Pr_{(before)}\{D_i\}\frac{1 - P_{ij}}{1 - S_j}$$

where $Pr_{(before)}\{D_i\}$ is the existence probability of disease Di before updating, $P_{ij}$ is the conditional probability that Symptom j indicates disease i, $S_j$ is the unconditional probability of Symptom j;

q. identifying with the processor a qualifying disease in each list of diseases with said updated existence probability greater than a predetermined threshold value, adding each Symptom associated to said qualifying disease to the first iterative dataset (S) if the Symptom to be added is not already in the first iterative dataset (S) or in the second iterative dataset (Q), and deleting the target Symptom from the first iterative dataset (S) and adding the target symptom and the fact of its existence or non existence according to the patient's answer to the second iterative dataset (Q);

r. repeating steps n through q above until each Symptom contained in the first iterative dataset (S) is deleted; and

s. generating and displaying on a display device a pre-diagnosis report using the second iterative dataset (Q) by using the processor to identify a list of diseases and the existence probability of each disease in the list, including the identity of a most probable disease having the highest probability of existence.

2. The interactive computer system according to claim 1, wherein in item "s" the generated list of diseases further includes a sublist containing each probable disease having a probability of existence in excess of a predetermined amount.

3. The interactive computer system according to claim 1, further adapted for performing the steps as described in t to w :

   t. displaying the pre-diagnosis report to a patient's nurse on a nurse display device whereby the nurse may review the patient's vitals, medical history, environmental exposures and Symptoms to determine whether the information inputted by the patient is accurate and whether the patient has adequately responded to the computer generated interview questions which identify the patient's Symptoms;
   u. displaying the pre-diagnosis report to a patient's physician on a physician display device whereby the physician may review the report to obtain medical information about the patient and the patient's condition, including information about the probable existence of diseases related to the patient's medical information and condition;
   v. prompting the patient's physician to confirm, by using the physician data entry device in communicating with the processor, that the doctor's own diagnosis of the patient's actual disease is the same as the most probable disease described in the pre-diagnosis report;
   w. updating the existence probability of said most probable disease by using the processor to increment the disease numerical value of said most probable disease by one (1) if the patient's physician confirms that the most probable disease is his or her actual disease.

4. The interactive computer system according to claim 2, further adapted for performing the steps "t" and "u" of claim 3 and further adapted for performing the steps v and w :

   v. prompting the patient's physician to confirm, by using the physician data entry device in communicating with the processor, that the doctor's own diagnosis of the patient's actual disease is the same as the most probable disease or one of the probable diseases in the sublist described in the pre-diagnosis report;
   w. updating the existence probability of said one probable disease by using the processor to increment the disease numerical value of said one probable disease by one (1) if the patient's physician confirms that the probable disease is his or her actual disease..

5. The interactive computer system according to claim 1, adapted for performing the steps "t" and "u" of claim 3 and further, adapted for performing the steps v to z :

   v. prompting the patient's physician to indicate, by using the physician data entry device in communication with the processor, that the patient exhibits a condition revealed in laboratory tests;
   w. calculating the updated existence probability Pr {D|S} of each disease in each list of diseases based upon the physician's indication that the condition exists in accordance with the following formula:

$$\Pr\{D \mid S\} = \Pr\{D\}\frac{\Pr\{S \mid D\}}{\Pr\{S\}}$$

   where D is a disease and S is a Symptom;
   x. generating a revised pre-diagnosis report for the physician including a list of diseases and a revised existence probability of each disease in the list, including the identity of a most probable disease having the highest probability of existence;
   y. prompting the patient's physician to confirm by using the physician data entry device in communicating with the processor, that the doctor's own diagnosis of the patient's actual disease is the same as the most probable disease described in the revised pre-diagnosis report;
   z. updating the existence probability of said one probable disease by using the processor to increment the disease numerical value of said most probable disease by one (1) if the patient's physician confirms that the most probable disease is his or her actual disease.

6. The interactive computer system according to claim 2, adapted for performing the steps "t" and "u" of claim 3, items "v" and "w" of claim 5 and further adapted for performing the steps x to z :

x. generating a revised pre-diagnosis report for the physician including a list of diseases and a revised existence probability of each disease in the list, including the identity of each probable disease having a probability in excess of predetermined amounts;

y. prompting the patient's physician to confirm by using the physician data entry device in communicating with the processor, that the doctor's own diagnosis of the patient's actual disease is the same as one of the probable diseases described in the revised pre-diagnosis report;

z. updating the existence probability of said one probable disease by using the processor to increment the disease numerical value of said one probable disease by one (1) if the patient's physician confirms that the probable disease is his or her actual disease.

7. A computer program product for use with a pre-diagnosis report generator, said computer program product comprising:

a. computer readable program code for prompting a patient to enter his or her vitals into the memory of a database server by using a patient data entry device in communication with the processor and similarly prompting the patient to enter the patient's medical history, family medical history and exposure to environmental conditions into the database server;

b. computer readable program code for accessing a list of Body Parts stored in the memory of the database server;

c. computer readable program code for accessing in the memory of the database server the lists of diseases and lists of Symptoms according to claim 1, step f;

d. computer readable program code for accessing in said memory the disease numerical values according to claim 1, step g;

e. computer readable program code for accessing in said memory the symptom numerical values according to claim 1, step h;

f. computer readable program code for identifying with the processor sets of Symptoms $(S_1,...,S_p)$, and each Symptom's associated symptom numerical value, such that for each stored Body Part a set of Symptoms $(S_1,...,S_p)$ is identified in association with that Body Part, said set of Symptoms comprising all Symptoms associated to that Body Part; and

comprising computer readable program code, when run on an interactive computer system according to claim 1, items a, b and c, wherein stored in the memory of the database server lists and values according to claim 1, steps e to h, causing said interactive computer system to carry out either one of:

steps "j" to "s" as defined in claim 1 or 2
steps "j" to "w" as defined in claim 3 or 4
steps "j" to "z" as defined in claim 5 or 6.

8. A method of operating an interactive computer system as defined in claim 1 items "a", "b" and "c" comprising method steps "d" to "s" as defined in claim 1.

**Patentansprüche**

1. Interaktives Computersystem umfassend:

a. einen programmierten Computerprozessor;

b. einen Datenbankserver mit einem Speicher, wobei der Datenbankserver in elektronischer Verbindung mit dem Prozessor steht;

c. eine Mehrzahl von Patientendateneingabe- und -anzeigegeräten, die alle in elektronischer Verbindung mit dem Prozessor stehen; dafür geeignet die Schritte wie in d bis s beschrieben durchzuführen:

d. Auffordern eines Patienten aus einer Mehrzahl von Patienten seine oder ihre Vitalwerte in den Speicher des Datenbankservers einzugeben durch Verwenden eines der Patientendateneingabegeräte, das in Kommunikation mit dem Prozessor steht, und um gleichermaßen die persönliche medizinische Geschichte, familiäre medizinische Geschichte und eine Aussetzung gegenüber Umweltbedingungen des Patienten in den Datenbankserver einzugeben;

e. Speichern einer Liste von Körperteilen im Speicher des Datenbankservers;

f. Speichern von Listen von Krankheiten ($Krankheit_1$, ... $Krankheit_m$) im Speicher, so dass für jeden gespeicherten Körperteil eine Liste von Krankheiten, die wenigstens eine Krankheit umfasst, in Zuordnung zu dem gespeicherten Körperteil gespeichert ist, und von Listen von Symptomen im Speicher (${Symptom_{1(1)}, ... ,}$

Symptom$_{1(n)}$},...,{Symptom$_{m(1)}$}), ... , Symptom$_{m(n)}$}), so dass für jede gespeicherte Krankheit aus einer der gespeicherten Listen von Krankheiten eine Liste von Symptomen in Zuordnung zu dieser Krankheit gespeichert ist;

g. Speichern von numerischen Krankheitswerten (DPC$_1$, ... , DPC$_m$) im Speicher, so dass für jede gespeicherte Krankheit aus einer der gespeicherten Listen von Krankheiten ein numerischer Krankheitswert in Zuordnung zu dieser Krankheit gespeichert ist, wobei jeder numerische Krankheitswert einen Wert gleich der Anzahl der Personen besitzt, bei denen bestätigt wurde, diese spezifische Krankheit zu haben;

h. Speichern von numerischen Symptomwerten (SPC$_1$, ... , SPC$_m$) im Speicher, so dass für jedes gespeicherte Symptom aus einer der Listen von Symptomen ein numerischer Symptomwert in Zuordnung zu diesem Symptom gespeichert ist, wobei jeder numerische Symptomwert, der in Zuordnung zu einem der Symptome in einer der Listen von Symptome gespeichert ist, die in Zuordnung zu einer der Krankheit gespeichert sind, die einen Wert gleich dem numerischen Krankheitswert (DPC$_1$, ... , DPC$_m$) dieser gleichen Krankheit aufweist;

i. Identifizieren von Mengen von Symptomen (S$_1$, ... , S$_p$) und des numerischen Symptomwerts, der jedem Symptom zugeordnet ist, mit dem Prozessor, so dass für jeden gespeicherten Körperteil eine Menge von Symptomen (S$_1$, ... , S$_p$) in Zuordnung zu dem Körperteil identifiziert wird, wobei die Menge der Symptome alle dem Körperteil zugeordneten Symptome umfasst,

j. Berechnen der Wahrscheinlichkeit des Vorhandenseins jeder Krankheit in jeder Liste von Krankheiten durch Verwenden des Prozessors, um den Quotienten des numerischen Krankheitswerts von jeder Krankheit geteilt durch die Summe aller numerischen Krankheitswerte für alle Krankheiten zu bestimmen, und Speichern des Ergebnisses im Speicher des Datenbankservers;

k. Berechnen eines numerischen Gesamtsymptomwerts für alle identischen Symptome in jeder Liste von Symptomen durch Verwenden des Prozessors, um die Summe aller numerischen Symptomwerte für alle identischen Symptome in jeder Liste zu bestimmen, und Speichern des Ergebnisses im Speicher des Datenbankservers;

l. Anzeigen einer menschlichen Figur dem Patienten auf einem der Patientenanzeigegeräte, die eine Mehrzahl von Körperteilen aufweist;

m. Auffordern des Patienten unter Verwendung des Patienteneingabegeräts ein Zielkörperteil aus allen angezeigten Körperteilen zu identifizieren, von dem der Patient glaubt, dass es in Zuordnung zu seiner oder ihrer körperlichen oder mentalen Verfassung steht, und Definieren eines ersten iterativen Datensatzes (S), der anfangs die Menge aller Symptome ist, die dem Zielkörperteil zugeordnet ist, und Definieren eines zweiten iterativen Datensatzes (Q), der anfangs leer ist;

n. Identifizieren eines Zielsymptoms mit dem Prozessor aus dem ersten iterativen Datensatz (S), der den höchsten numerischen Gesamtsymptomwert aufweist, und Generieren einer Frage, die dem Patienten auf dem Patientenanzeigegeräte präsentiert wird, das den Patienten fragt, ob das Zielsymptom angezeigt wird, oder nicht;

o. Auffordern des Patienten die Frage unter Verwendung des Patienteneingabegeräts zu beantworten;

p. Berechnen einer aktualisierten Wahrscheinlichkeit des Vorhandenseins Pr$_{(nachher)}${D$_1$} jeder Krankheit (D$_1$) in jeder Liste von Krankheiten basierend auf der Antwort des Patienten durch Verwenden des Prozessors, um die Wahrscheinlichkeit des Vorhandenseins gemäß der folgenden Formel gemäß Bayesscher Statistik zu bestimmen:

$$Pr\{D|S\}=Pr\{D\}\frac{Pr\{S|D\}}{Pr\{S\}}$$

wobei D eine Krankheit und S ein Symptom ist, Pr{...} eine nicht bedingte Wahrscheinlichkeit und Pr{...|...} eine bedingte Wahrscheinlichkeit ist, und zwar durch Berechnen der aktualisierten Wahrscheinlichkeit des Vorhandenseins, falls der Patient anzeigt, dass er/sie das Symptom hat, unter Verwendung der Gleichung:

$$Pr_{(nachher)}\{D_i\}=Pr_{(vorher)}\{D_i\}\frac{P_{ij}}{S_j}$$

und falls der Patient anzeigt, dass er/sie das Symptom nicht hat, unter Verwendung der Gleichung:

$$Pr_{(nachher)}\{D_i\} = Pr_{(vorher)}\{D_i\} \frac{1 - P_{ij}}{1 - S_j}$$

wobei $Pr_{(vorher)}\{D_1\}$ die Wahrscheinlichkeit des Vorhandenseins der Krankheit $D_i$ vor dem Aktualisieren ist, $P_{ij}$ die bedingte Wahrscheinlichkeit ist, dass Symptom j die Krankheit i anzeigt, $S_j$ die nicht bedingte Wahrscheinlichkeit des Symptoms j ist;

q. Identifizieren einer qualifizierenden Krankheit mit dem Prozessor in jeder Liste von Krankheiten mit der aktualisierten Wahrscheinlichkeit des Vorhandenseins, die größer als ein vorbestimmter Grenzwert ist, Hinzufügen jedes Symptoms, das der qualifizierenden Krankheit zugeordnet ist, zum ersten iterativen Datensatz (S), falls das hinzuzufügende Symptom sich noch nicht im ersten iterativen Datensatz (S) oder im zweiten iterativen Datensatz (Q) befindet, und Löschen des Zielsymptoms aus dem ersten iterativen Datensatz (S) und Hinzufügen des Zielsymptoms und der Tatsache seines Vorhandenseins oder Nicht-Vorhandenseins gemäß der Antwort des Patienten zum zweiten iterativen Datensatz (Q);

r. Wiederholen der obigen Schritte n bis q bis jedes Symptom, das im ersten iterativen Datensatz (S) enthalten ist, gelöscht ist; und

s. Erzeugen und Anzeigen eines prädiagnostischen Berichts auf einem Anzeigegerät, der den zweiten iterativen Datensatz (Q) verwendet, durch Verwendung des Prozessors, um eine Liste von Krankheiten und die Wahrscheinlichkeit des Vorhandenseins jeder der Krankheiten in der Liste zu identifizieren, und der die Identität der wahrscheinlichsten Krankheit mit der höchsten Wahrscheinlichkeit des Vorhandenseins umfasst.

**2.** Interaktives Computersystem nach Anspruch 1,
wobei in Punkt "s" die erzeugte Liste von Krankheiten weiter eine Unterliste umfasst, die jede wahrscheinliche Krankheit enthält, die eine Wahrscheinlichkeit des Vorhandenseins größer als einen vorbestimmten Wert aufweist.

**3.** Interaktives Computersystem nach Anspruch 1,
weiter geeignet zum Durchführen der in t bis w beschriebenen Schritte:

t. Anzeigen des prädiagnostischen Berichts einer Pflegekraft des Patienten auf einem Pflegekraftanzeigegeerät, wobei die Pflegekraft die Vitalwerte, die medizinische Geschichte, die Aussetzung gegenüber Umweltbedingungen und Symptome des Patienten durchsehen kann, um zu bestimmen, ob die vom Patienten eingegebenen Informationen genau sind und ob der Patient auf die vom Computer erzeugten Interviewfragen ausreichend geantwortet hat, die die Symptome des Patienten identifizieren;

u. Anzeigen des prädiagnostischen Berichts einem Arzt des Patienten auf einem Arztanzeigegerät, wobei der Arzt den Bericht durchsehen kann, um medizinische Informationen über den Patienten und die Verfassung des Patienten zu erhalten, umfassend Informationen über das wahrscheinliche Vorhandensein von Krankheiten, die mit den medizinischen Informationen und der Verfassung des Patienten in Beziehung stehen;

v. Auffordern des Arztes des Patienten unter Verwendung des Arztdateneingabegeräts, das in Verbindung mit dem Prozessor steht, zu bestätigen, dass die eigene Diagnose des Arztes der tatsächlichen Krankheit des Patienten die gleiche ist, wie die wahrscheinlichste Krankheit, die im prädiagnostischen Bericht beschrieben ist;

w. Aktualisieren der Wahrscheinlich des Vorhandenseins der wahrscheinlichsten Krankheit durch Verwenden des Prozessors, um den numerischen Krankheitswert der wahrscheinlichsten Krankheit um Eins (1) zu erhöhen, wenn der Arzt des Patienten bestätigt, dass die wahrscheinlichste Krankheit seine oder ihre tatsächliche Krankheit ist.

**4.** Interaktives Computersystem nach Anspruch 2,
weiter geeignet zum Durchführen der Schritte "t" und "u" aus Anspruch 3 und weiter geeignet zum Durchführen der Schritte "v" und "w":

v. Auffordern des Arztes des Patienten unter Verwendung des Arztdateneingabegeräts, das in Verbindung mit dem Prozessor steht, zu bestätigen, dass die eigene Diagnose des Arztes der tatsächlichen Krankheit des Patienten die gleiche ist, wie die wahrscheinlichste Krankheit oder eine der wahrscheinlichen Krankheiten in der Unterliste, die im prädiagnostischen Bericht beschrieben ist;

w. Aktualisieren der Wahrscheinlichkeit des Vorhandenseins der einen wahrscheinlichen Krankheit durch Verwenden des Prozessors, um den numerischen Krankheitswert der einen wahrscheinlichen Krankheit um Eins (1) zu erhöhen, wenn der Arzt des Patienten bestätigt, dass die wahrscheinliche Krankheit seine oder ihre tatsächliche Krankheit ist.

**5.** Interaktives Computersystem nach Anspruch 1,
weiter geeignet zum Durchführen der Schritte "t" und "u" aus Anspruch 3 und weiter geeignet zum Durchführen der Schritte "v" bis "z":

v. Auffordern des Arztes des Patienten unter Verwendung des Arztdateneingabegeräts, das in Verbindung mit dem Prozessor steht, anzugeben, dass der Patient eine Verfassung aufweist, die in Labortest offenbart wurde;
w. Berechnen der aktualisierten Wahrscheinlichkeit des Vorhandenseins Pr{D|S} jeder Krankheit in jeder Liste von Krankheiten basierend auf der Angabe des Arztes, dass die Verfassung vorhanden ist, gemäß der folgenden Formel:

$$Pr\{D|S\} = Pr\{D\}\frac{Pr\{S|D\}}{Pr\{S\}}$$

wobei D eine Krankheit ist und S ein Symptom ist;

x. Erzeugen eines revidierten prädiagnostischen Berichts für den Arzt, der eine Liste von Krankheiten und eine revidierte Wahrscheinlichkeit des Vorhandenseins jeder Krankheit in der Liste umfasst, der die Identität der wahrscheinlichsten Krankheit mit der höchsten Wahrscheinlichkeit des Vorhandenseins umfasst;
y. Auffordern des Arztes des Patienten unter Verwendung des Arztdateneingabegeräts, das in Verbindung mit dem Prozessor steht, zu bestätigen, dass die eigene Diagnose des Arztes der tatsächlichen Krankheit des Patienten die gleiche ist, wie die wahrscheinlichste Krankheit, die im revidierten prädiagnostischen Bericht beschrieben ist;
z. Aktualisieren der Wahrscheinlichkeit des Vorhandenseins der einen wahrscheinlichen Krankheit durch Verwenden des Prozessors, um den numerischen Krankheitswert der wahrscheinlichsten Krankheit um Eins (1) zu erhöhen, wenn der Arzt des Patienten bestätigt, dass die wahrscheinlichste Krankheit seine oder ihre tatsächliche Krankheit ist.

**6.** Interaktives Computersystem nach Anspruch 2,
weiter geeignet zum Durchführen der Schritte "t" und "u" aus Anspruch 3, der Schritte "v" und "w" aus Anspruch 5 und weiter geeignet zum Durchführen der Schritte "x" bis "z":

x. Erzeugen eines revidierten prädiagnostischen Berichts für den Arzt, der eine Liste von Krankheiten und eine revidierte Wahrscheinlichkeit des Vorhandenseins jeder Krankheit in der Liste umfasst, der die Identität jeder wahrscheinlichen Krankheit mit einer Wahrscheinlichkeit größer als vorbestimmte Werte umfasst;
y. Auffordern des Arztes des Patienten unter Verwendung des Arztdateneingabegeräts, das in Verbindung mit dem Prozessor steht, zu bestätigen, dass die eigene Diagnose des Arztes der tatsächlichen Krankheit des Patienten die gleiche ist, wie eine der wahrscheinlichen Krankheiten, die im revidierten prädiagnostischen Bericht beschrieben sind;
z. Aktualisieren der Wahrscheinlichkeit des Vorhandenseins der einen wahrscheinlichen Krankheit durch Verwenden des Prozessors, um den numerischen Krankheitswert der einen wahrscheinlichen Krankheit um Eins (1) zu erhöhen, wenn der Arzt des Patienten bestätigt, dass die wahrscheinliche Krankheit seine oder ihre tatsächliche Krankheit ist.

**7.** Computerprogrammprodukt zur Verwendung mit einem prädiagnostischen Berichterzeuger, wobei das Computerprogrammprodukt umfasst:

a. computerlesbaren Programmcode zum Auffordern eines Patienten, seine oder ihre Vitalwerte in den Speicher eines Datenbankservers durch Verwenden eines Patientendateneingabegeräts, das in Verbindung mit dem Prozessor steht, einzugeben, und zum gleichermaßen Auffordern des Patienten die medizinische Geschichte, die familiäre medizinische Geschichte und die Aussetzung gegenüber Umweltbedingungen des Patienten in den Datenbankserver einzugeben;
b. computerlesbaren Programmcode zum Zugreifen auf eine Liste von Körperteilen, die im Speicher des Datenbankservers gespeichert ist;
c. computerlesbaren Programmcode zum Zugreifen im Speicher des Datenbankservers auf die Listen von Krankheiten und Listen von Symptomen nach Anspruch 1, Schritt f;
d. computerlesbaren Programmcode zum Zugreifen im Speicher auf die numerischen Krankheitswerte nach Anspruch 1, Schritt g;

e. computerlesbaren Programmcode zum Zugreifen im Speicher auf die numerischen Symptomwerte nach Anspruch 1, Schritt h;

f. computerlesbaren Programmcode zum Identifizieren von Mengen von Symptomen ($S_1$, ... , $S_p$) und des numerischen Symptomwerts, der jeden Symptom zugeordnet ist, mit dem Prozessor, so dass für jeden gespeicherten Körperteil eine Menge von Symptomen ($S_1$, ... , $S_p$) in Zuordnung zu jenem Körperteil identifiziert wird, wobei die Menge der Symptome alle dem Körperteil zugeordneten Symptome umfasst; und umfassend computerlesbaren Programmcode, der, wenn er auf einem interaktiven Computersystem nach Anspruch 1, Punkte a, b und c ausgeführt wird, wobei im Speicher des Datenbankservers Listen und Werte nach Anspruch 1, Schritte e bis h gespeichert sind, bewirkt, dass das interaktive Computersystem eines des Folgenden ausführt:

die wie in Anspruch 1 oder 2 definierten Schritte "j" bis "s",
die wie in Anspruch 3 oder 4 definierten Schritte "j" bis "w",
die wie in Anspruch 5 oder 6 definierten Schritte "j" bis "z",

8. Verfahren zum Betrieb eines interaktiven Computersystems definiert wie in Anspruch 1, Punkte "a", "b" und "c" umfassend die wie in Anspruch 1 definierten Verfahrensschritte "d" bis "s".

**Revendications**

1. Un système informatique interactif comprenant :

a. un processeur d'ordinateur programmé ;

b. un serveur de base de données ayant une mémoire, ledit serveur de base de données en communication électronique avec le processeur ;

c. une pluralité de dispositifs de saisie de données et d'affichage pour patient, tous en communication électronique avec le processeur ; adaptés pour exécuter les étapes comme décrit dans d à s :

d. inciter un patient, parmi une pluralité de patients, à entrer ses paramètres vitaux dans la mémoire du serveur de base de données en employant un desdits dispositifs de saisie de données pour patient en communication avec le processeur et à entrer pareillement les antécédents médicaux personnels du patient, des antécédents médicaux de famille et l'exposition aux conditions environnementales dans le serveur de base de données ;

e. stocker une liste de Parties Corporelles dans la mémoire du serveur de base de données ;

f. stocker dans ladite mémoire, des listes de maladies ($Disease_1$,..,$Disease_m$) telles que pour chaque Partie Corporelle stockée, une liste de maladies comportant au moins une maladie est stockée en association avec cette Partie Corporelle stockée, et des listes de Symptômes {$Symptom_{1(1)}$,...,$Symptom_{1(n)}$} ,..,{$Symptom_{m(1)}$,...,$Symptom_{m(n)}$}) telles que pour chaque maladie stockée parmi chacune des listes stockées de maladies, une liste de Symptômes est stockée en association avec cette maladie ;

g. stocker dans ladite mémoire, des valeurs numériques de maladie ($DPC_1$, ..., $DPC_m$) telles que pour chaque maladie stockée de chacune des listes stockées de maladies, une valeur numérique de maladie est stockée en association avec cette maladie, chacune des dites valeurs numériques de maladie ayant une valeur égale au nombre de personnes qui ont été confirmées présentant cette maladie spécifique ;

h. stocker dans ladite mémoire, des valeurs numériques de Symptôme ($SPC_1$,...,$SPC_m$) telles que pour chaque Symptôme stocké parmi chacune des listes de Symptômes, une valeur numérique de Symptôme est stockée en association avec ce Symptôme, chacune des dites valeurs numériques de Symptôme qui est stockée en association avec un des dits Symptômes dans une des dites listes de Symptômes stockée en association avec une des dites maladies ayant une valeur égale à la valeur numérique de maladie ($DPC_1$, ..., $DPC_m$) de cette même dite maladie ;

i. identifier avec le processeur des ensembles de Symptômes ($S_1$,...,$S_p$), et la valeur numérique de Symptôme associée à chaque Symptôme, tels que pour chaque Partie Corporelle stockée un ensemble de Symptômes ($S_1$,...,$S_p$) est identifié en association avec cette Partie Corporelle, ledit ensemble de Symptômes comportant tous les Symptômes associés à cette Partie Corporelle ;

j. calculer la probabilité d'existence de chaque maladie dans chaque liste de maladies en employant le processeur pour déterminer le quotient de la valeur numérique de maladie de chaque maladie divisée par la somme de toutes les valeurs numériques de maladie pour toutes les maladies et stocker les résultats dans la mémoire du serveur de base de données ;

k. calculer une valeur numérique totale de Symptôme pour tous les Symptômes identiques dans chaque liste de Symptômes en employant le processeur pour déterminer la somme de toutes les valeurs numériques de Symptôme pour tous les Symptômes identiques dans chaque liste, et stocker les résultats dans la mémoire du serveur de base de données ;

l. montrer au patient sur un des dits dispositifs d'affichage pour patient une représentation humaine pourvue d'une pluralité de Parties Corporelles ;

m. inciter le patient à identifier, à l'aide du dispositif de saisie de données pour patient, une Partie Corporelle cible, parmi toutes les Parties Corporelles montrées, que le patient croit être associée à son état physique ou mental, et définir un premier ensemble de données itératif (S) étant initialement l'ensemble de tous les Symptômes associés à la Partie Corporelle cible, et définir un deuxième ensemble de données itératif (Q) étant initialement vide ;

n. identifier avec le processeur un Symptôme cible parmi le premier ensemble de données itératif (S) qui a la valeur numérique totale de Symptôme la plus élevée et générer une question présentée au patient sur le dispositif d'affichage pour patient qui demande au patient si le Symptôme cible est ou n'est pas exhibé ;

o. inciter le patient à répondre à la question à l'aide du dispositif de saisie de données pour patient ;

p. calculer une probabilité d'existence révisée $\text{Pr}_{(after)}\{D_i\}$ de chaque maladie $(D_i)$ dans chaque liste de maladie basée sur la réponse du patient en employant le processeur pour déterminer ladite probabilité d'existence selon la formule suivante selon des statistiques bayésiennes :

$$\text{Pr}\{D \mid S\} = \text{Pr}\{D\} \frac{\text{Pr}\{S \mid D\}}{\text{Pr}\{S\}}$$

où D est une maladie et S est un Symptôme, Pr{...} est une probabilité sans conditions et Pr{...|...} est une probabilité conditionnelle, en calculant la probabilité d'existence révisée au cas où le patient indiquerait qu'il/elle a le Symptôme, en utilisant l'équation :

$$\text{Pr}_{(after)}\{D_i\} = \text{Pr}_{(before)}\{D_i\} \frac{P_{ij}}{S_j}$$

et au cas où le patient indiquerait qu'il/elle n'a pas le Symptôme, en utilisant l'équation :

$$\text{Pr}_{(after)}\{D_i\} = \text{Pr}_{(before)}\{D_i\} \frac{1 - P_{ij}}{1 - S_j}.$$

où $\text{Pr}_{(before)}\{D_i\}$ est la probabilité d'existence de la maladie $D_i$ avant la révision, $P_{ij}$ est la probabilité conditionnelle que le Symptôme j indique la maladie i, $S_j$ est la probabilité sans conditions du Symptôme j ;

q. identifier avec le processeur une maladie qualifiée dans chaque liste de maladies dont ladite probabilité d'existence révisée est plus grande qu'une valeur-seuil prédéterminée, ajoutant chaque Symptôme associé à ladite maladie qualifiée au premier ensemble de données itératif (S) si le Symptôme à ajouter n'est pas déjà dans le premier ensemble de données itératif (S) ou dans le deuxième ensemble de données itératif (Q), et supprimer le Symptôme cible du premier ensemble de données itératif (S) et ajouter le Symptôme cible et le fait de son existence ou non-existence selon la réponse du patient au deuxième ensemble de données itératif (Q) ;

r. répéter les étapes n à q ci-dessus jusqu'à ce que chaque Symptôme contenu dans le premier ensemble de données itératif (S) soit supprimé ; et

s. produire et montrer sur un dispositif d'affichage un rapport de pré-diagnostic en utilisant le deuxième ensemble de données itératif (Q) en employant le processeur pour identifier une liste de maladies et la probabilité d'existence de chaque maladie dans la liste, y compris l'identité d'une maladie la plus probable ayant la probabilité d'existence la plus élevée.

2. Le système informatique interactif selon la revendication 1, pour lequel à l'étape « s » la liste de maladies produite inclut par ailleurs une sous-liste contenant chaque maladie probable ayant une probabilité d'existence au-dessus d'une quantité prédéterminée.

**3.** Le système informatique interactif selon la revendication 1, en outre adapté pour exécuter les étapes comme décrit dans t à w :

t. montrer le rapport de pré-diagnostic à l'infirmière d'un patient sur un dispositif d'affichage de l'infirmière au moyen duquel l'infirmière peut passer en revue les paramètres vitaux du patient, les antécédents médicaux, les expositions environnementales et les Symptômes pour déterminer si les informations entrées par le patient sont correctes et si le patient a adéquatement répondu aux questions d'entrevue générées par ordinateur qui identifient les Symptômes du patient ;

u. montrer le rapport de pré-diagnostic au médecin d'un patient sur un dispositif d'affichage du médecin au moyen duquel le médecin peut passer en revue le rapport afin d'obtenir des informations médicales sur le patient et l'état du patient, y compris des informations sur l'existence probable de maladies liées aux informations médicales sur le patient et à l'état du patient ;

v. inciter le médecin du patient à confirmer, en utilisant le dispositif de saisie de données du médecin en communication avec le processeur, que le propre diagnostic de la maladie réelle du patient par le docteur est identique à la maladie la plus probable décrite dans le rapport de pré-diagnostic ;

w. réviser la probabilité d'existence de la maladie la plus probable en employant le processeur pour incrémenter la valeur numérique de maladie de ladite maladie la plus probable par un (1) si le médecin du patient confirme que la maladie la plus probable est sa maladie réelle.

**4.** Le système informatique interactif selon la revendication 2, en outre adapté pour exécuter les étapes « t » et « u » de la revendication 3 et en outre adapté pour exécuter les étapes v et w ;

v. inciter le médecin du patient à confirmer, en utilisant le dispositif de saisie de données du médecin en communication avec le processeur, que le propre diagnostic par le docteur de la maladie réelle du patient est identique à la maladie la plus probable ou une des maladies probables dans la sous-liste décrite dans le rapport de pré-diagnostic ;

w. réviser la probabilité d'existence de ladite une maladie probable en employant le processeur pour incrémenter la valeur numérique de maladie de ladite une maladie probable par un (1) si le médecin du patient confirme que la maladie probable est sa maladie réelle.

**5.** Le système informatique interactif selon la revendication 1, adapté pour exécuter les étapes « t » et « u » de la revendication 3 et en outre adapté pour exécuter les étapes v à z :

v. inciter le médecin du patient à indiquer, en utilisant le dispositif de saisie de données du médecin en communication avec le processeur, que le patient présente une affection révélée par des essais en laboratoire ;

w. calculer la probabilité d'existence révisée Pr {DIS} de chaque maladie dans chaque liste de maladies basée sur l'indication du médecin que l'affection existe, selon la formule suivante :

$$\Pr\{D \mid S\} = \Pr\{D\}\frac{\Pr\{S \mid D\}}{\Pr\{S\}}$$

où D est une maladie et un S est un Symptôme ;

x. produire un rapport de pré-diagnostic révisé pour le médecin comprenant une liste de maladies et une probabilité d'existence révisée de chaque maladie dans la liste, y compris l'identité d'une maladie la plus probable ayant la probabilité d'existence la plus élevée ;

y. inciter le médecin du patient à confirmer en utilisant le dispositif de saisie de données du médecin en communication avec le processeur, que le propre diagnostic du docteur de la maladie réelle du patient est identique à la maladie la plus probable décrite dans le rapport révisé de pré-diagnostic ;

z. réviser la probabilité d'existence de ladite une maladie probable en employant le processeur pour incrémenter la valeur numérique de maladie de la maladie la plus probable par un (1) si le médecin du patient confirme que la maladie la plus probable est sa maladie réelle.

**6.** Le système informatique interactif selon la revendication 2, adapté pour exécuter les étapes « t » et « u » de la revendication 3, les étapes « v » et « w » de la revendication 5 et en outre adapté pour exécuter les étapes x à z :

x. produire un rapport de pré-diagnostic révisé pour le médecin comprenant une liste de maladies et une probabilité d'existence révisée de chaque maladie dans la liste, y compris l'identité de chaque maladie probable ayant une probabilité au-dessus de quantités prédéterminées ;

y. inciter le médecin du patient à confirmer en utilisant le dispositif de saisie de données du médecin en communication avec le processeur, que le propre diagnostic par le docteur de la maladie réelle du patient est identique à l'une des maladies probables décrites dans le rapport révisé de pré-diagnostic ;

z. réviser la probabilité d'existence de ladite une maladie probable en employant le processeur pour incrémenter la valeur numérique de maladie de ladite une maladie probable par un (1) si le médecin du patient confirme que la maladie probable est sa maladie réelle.

7. Un produit de programme informatique pour l'usage avec un générateur de rapport de pré-diagnostic, ledit de produit de programme informatique comprenant :

a. du code de programme informatique lisible par ordinateur pour inciter un patient à entrer ses paramètres vitaux dans la mémoire d'un serveur de base de données en utilisant une unité de saisie de données pour patient en communication avec le processeur et inciter pareillement le patient à écrire les antécédents médicaux du patient, des antécédents médicaux de famille et l'exposition aux conditions environnementales dans le serveur de base de données ;

b. du code de programme informatique lisible par ordinateur pour accéder à une liste de Parties Corporelles stockées dans la mémoire du serveur de base de données ;

c. du code de programme informatique lisible par ordinateur pour accéder dans la mémoire du serveur de base de données aux listes de maladies et à des listes de Symptômes selon la revendication 1, étape f ;

d. du code de programme informatique lisible par ordinateur pour accéder dans ladite mémoire aux valeurs numériques de maladie selon la revendication 1, étape g ;

e. du code de programme informatique lisible par ordinateur pour accéder dans ladite mémoire aux valeurs numériques de Symptôme selon la revendication 1, étape h ;

f. du code de programme informatique lisible par ordinateur pour identifier avec le processeur des ensembles de Symptômes $(S_1,...,S_p)$, et la valeur numérique de Symptôme associée à chaque Symptôme, telle que pour chaque Partie Corporelle stockée un ensemble de Symptômes $(S_1,...,S_p)$ est identifié en association avec cette Partie Corporelle, lequel ensemble de Symptômes comprend tous les Symptômes associés à cette Partie Corporelle ; et comprenant du code de programme informatique lisible par ordinateur, qui une fois exécuté sur un système informatique interactif selon la revendication 1, aux points a, b et c, dans lequel sont stockées dans la mémoire du serveur de base de données des listes et des valeurs selon la revendication 1, étapes e à h, fait effectuer par ledit système informatique interactif l'un ou l'autre parmi :

les étapes « j » à « s » comme définies dans la revendication 1 ou 2
les étapes « j » à « w » comme définies dans la revendication 3 ou 4
les étapes « j » à « z » comme définies dans la revendication 5 ou 6.

8. Une méthode pour actionner un système informatique interactif comme défini dans la revendication 1, aux points « a », « b » et « c » comprenant les étapes de méthode « d » à « s » comme définies dans la revendication 1.

Fig.1

**DATABASE SERVER** 3

PATIENT'S PERSONAL INFORMATION

MEDICAL HISTORY
- VITALS
- PERSONAL
- FAMILY
- ENVIRONMENT

DISEASES

SYMPTOMS

BODY PARTS

MULTIPLE PATIENTS 1

APPLICATION PROCESSOR 2

NURSE 4

PRE-DIAGNOSIS REPORT

PROBABLE DISEASES

LAB TESTS

SPECIALISTS

DRUGS

ALL QUESTIONS & ANSWERS

5

PHYSICIAN 6

PHYSICIAN EXAMINES PATIENT 7

PHYSICIAN ORDERS REPORTS 8

LAB TESTS REPORT

PHARMACEUTICAL REPORT 9

SPECIALIST'S REPORT

PHYSICIAN'S DIAGNOSIS 10

PHYSICIAN CONFIRMS PRE-DIAGNOSIS? 11

—Yes—

No

INCREMENT DISEASE COUNT & ADD DISEASE TO MEDICAL HISTORY 12

PHYSICIAN REVIEW 13

# "Disease/Symptom" Database

Fig.2

Fig.3

# Vitals

### Degree of Pain

- Off & On
- Constant
- Worst-ever
- Severe
- Moderate
- Light

### Temperature

- High
- Fever
- Normal
- Sub

### Weight

### Blood Pressure

Fig. 4

EP 1 284 639 B1

Fig.5

EP 1 284 639 B1

# Body Parts

- Skin
- Whole Body
- Chest
- Heart
- Lungs

- Shoulder

- Brain
- Eye
- Ear
- Nose
- Mouth
- Scalp
- Throat
- Neck

- Arm
- Hand
- Elbow
- Wrist
- Fingers

- Anus
- Vagina
- Penis
- Testicles
- Stomach

- Hip
- Knee
- Ankle
- Leg
- Thigh
- Calf

- Toes
- Arch
- Heel

Fig.6

.Fig.7

# QUESTION GENERATION TABLE

| QUESTION PHRASES | TEMPLATE CODES |
|---|---|
| DO OTHERS | 3 |
| DO YOU | 3 |
| DO YOU HAVE | 2 |
| DO YOU HAVE A | 3 |
| ARE YOU | 3 |
| IS YOUR | 1 |
| HAVE YOU | 2 |
| HAVE YOU A | 3 |
| ARE YOUR | 1 |
| DOES YOUR | 1 |
| WHAT IS YOUR | 3 |
| HOW OFTEN | 3 |
| IS IT | 3 |
| HAVE YOU HAD | 3 |

Fig.8

EP 1 284 639 B1

```
Obtain Question
Phrase for
Symptom
```

```
Obtain Template
Code for Question
Phrase
```

1

| Question Phrase | Body Part | Symptom |
|---|---|---|
| "Does your | head | ache?" |

2

| Question Phrase | Symptom | Body Part |
|---|---|---|
| "Do you have a | swollen | knee?" |

3

| Question Phrase | Symptom |
|---|---|
| "Do you have | diabetes?" |

Fig.9

```
┌─────────────────────────┐
│  Initialize "Disease/   │
│  Symptom" Database      │
│  (See FIG. 2)        1  │
└─────────────────────────┘
            │
            ▼
┌─────────────────────────┐
│  Let "S" be the set of all │
│  symptoms related to body  │
│  part selected by patient  │
│  (See FIG. 2)         2  │
└─────────────────────────┘
            │
            ▼
┌─────────────────────────┐
│  Let "Q" be an empty set │
│                       3  │
└─────────────────────────┘
            │
            ▼
┌─────────────────────────┐
│  Select symptom from set │
│  "S" with highest frequency │
│  (person count)          │
│                       4  │
└─────────────────────────┘
            │
            ▼
┌─────────────────────────┐
│  Generate symptom       │
│  question               │
│  (See FIGs. 5,6,7 & 8)  5│
└─────────────────────────┘
            │
            ▼
```

Does patient indicate "yes," "no," or "don't know" to existence of symptom? (See FIG. 6)    6

No

Yes/No

Update probable existence of all related diseases 7

Don't Know

Is "S" empty?    10

Remove symptom from "S" and add to "Q"    9

If any updated probability exceeds threshold (eg. 20%), add all related symptoms not in "Q" or "S" to "S"    8

Yes

Generate Pre-Diagnosis Report    11

Fig.10

## PREDIAGNOSIS REPORT

I.      Diseases ("ICD")      Probabilities

                _____           _____%

                _____           _____%

                _____           _____%

                _____           _____%

II.      Recommended Lab Tests

III.      Recommended Specialists

IV.      Questions & Answers and Medical History

V.      Confirm Prediagnosis

           Yes

           No

**EP 1 284 639 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5778882 A

- US 6022315 A